# FASCICULE DE BREVET EUROPEEN

(11) **EP 0 884 065 B1**
(45) Date de publication et mention de la délivrance du brevet: **17.01.2007**
(21) Numéro de dépôt: 98401373.0
(22) Date de dépôt: 09.06.1998
(51) Int. Cl.: A61N 1/362

(54) **Dispositif et procédé pour la détermination d'un critère de présence fréquente ou de gravité d'extra-systole (auriculaire ou ventriculaire)**
Gerät und Verfahren zur Bestimmung eines Kriteriums häufig auftretender oder gefährlicher Extrasystolen (atrial oder ventrikulär)
Device and method for determining a criterion of frequent occurance or severe extrasystoles (atrial or ventricular)

(30) Priorité: 09.06.1997 FR 9707115
(43) Date de publication de la demande: 16.12.1998
(73) Titulaire: ELA MEDICAL (Société anonyme), 92541 Montrouge (FR)
(72) Inventeur: Bonnet, Jean-Luc, 92120 Montrouge (FR); Bouhour, Anne, 92410 Ville d'Avray (FR)
(74) Mandataire: Dupuis-Latour, Dominique

(56) Documents cités:
- EP-A- 0 550 342
- FR-A- 2 717 397
- US-A- 5 423 863
- US-A- 5 513 645

## Description

L'invention concerne un dispositif médical implantable actif, en particulier un dispositif de la famille des stimulateurs, des défibrillateurs et des cardioverteurs chargés de délivrer au coeur des impulsions électriques, approprié à la détermination d'un critère de présence fréquente ou de gravité d'extrasystoles (auriculaire ou ventriculaire) ainsi qu'un procédé de détermination d'un critère de présence fréquente ou de gravité d'extrasystoles pour la commande d'un tel dispositif.

De tels dispositifs sont définis, par exemple, dans la directive 90/385/CEE du 20 juin 1990 du Conseil des communautés européennes.

On trouve parmi ces dispositifs des appareils dits "double chambre" dans la mesure où ils recueillent et délivrent des signaux dans les cavités haute (l'oreillette) et basse (le ventricule) du coeur.

Ces dispositifs sont connus pour suivre le rythme cardiaque du patient et effectuer certaines fonctions de diagnostic et/ou de thérapie des arythmies auriculaires (AA) et/ou ventriculaires (AV).

Ces fonctions sont exécutées de façon attendue tant que les arythmies (AA ou AV) présentent une forme prédéfinie (extrasystoles isolées, en salves, troubles du rythme) mais elles peuvent être prises en défaut par une alternance entre ces différentes formes.

La conséquence peut être un diagnostic inapproprié, avec éventuellement l'application d'une thérapie inadaptée voire dangereuse.

Généralement, on définit deux types d'extrasystoles ventriculaires (ESV).

Une extrasystole ventriculaire du premier type correspond à une détection ou stimulation ventriculaire non précédée d'un événement auriculaire (détection ou stimulation délivrée par le dispositif dans l'oreillette) dans un intervalle de temps considéré comme physiologique, par exemple compris entre 31 et 300 ms.

Une extrasystole ventriculaire du deuxième type correspond à une détection ventriculaire précédée d'un événement auriculaire dans un intervalle de temps compris entre 31 et 300 ms, dans le cas où le délai auriculo-ventriculaire (AR), du cycle examiné est inférieur de plus de 31 ms au délai auriculo-ventriculaire (DAV) du cycle cardiaque précédent (DAV-AR > 31 ms), le cycle cardiaque étant défini comme l'intervalle de temps entre deux événements de même nature dans la même cavité

Pour de plus amples détails sur le traitement des extrasystoles ventriculaires, on pourra se référer au document EP-A-0 550 342 décrivant un algorithme d'actions spécifiques à la suite de la détection d'une extrasystole ventriculaire.

Une onde ou un événement P (le recueil d'une activité spontanée ayant son origine dans l'oreillette) est défini comme une extrasystole auriculaire (ESA) si l'intervalle de temps séparant cette onde P du précédent événement auriculaire est inférieur à une fraction de l'intervalle moyen de la fréquence auriculaire calculée sur huit cycles cardiaques ne comprenant pas d'extrasystole (PP moyen).

De plus, dans la littérature, on trouve définis des doublets ou triplets d'extrasystoles qui sont, respectivement, une suite de deux extrasystoles (ESA ou ESV) sans événement non extrasystolique intermédiaire ou une suite de trois extrasystoles sans événement non extrasystolique intermédiaire.

Ces doublets ou triplets d'extrasystoles surviennent par séquences de quelques répétitions.

On connaît aussi des salves d'extrasystoles qui sont des répétitions très rapprochées d'extrasystoles (auriculaires ou ventriculaires) pouvant présenter de multiples événements sans événement non extrasystolique intermédiaire.

Ces salves d'extrasystoles sont des événements cardiaques à caractère inquiétant et leur présence est un signe de gravité du pronostic cardiaque du patient.

Les salves d'extrasystoles peuvent nécessiter la délivrance d'un choc de défibrillation si elles dégénèrent dans le ventricule en une fibrillation.

On définit aussi les troubles du rythme soutenus, comme étant des salves dont la durée est supérieure à un temps prédéfini (typiquement 30 s).

Le but de la présente invention est de proposer un procédé et un dispositif pour déterminer un critère de présence fréquente ou de gravité d'extrasystoles (auriculaires ou ventriculaires) isolées ou en salve en vue soit d'arrêter une fonction en cours, soit d'inhiber le déclenchement d'un autre type de fonction, soit au contraire, d'autoriser le déclenchement de fonctions spécifiques.

À cet effet le dispositif médical implantable actif selon l'invention comprend des moyens de détection des signaux d'activité cardiaque dans au moins une cavité cardiaque, des moyens de détermination du cycle cardiaque, des moyens de détection de la survenue d'extrasystoles un compteur d'extrasystoles, des moyens d'incrémentation du compteur d'extrasystoles à la détection de chaque extrasystole et des moyens de décrémentation à chaque cycle cardiaque du compteur d'extrasystoles en l'absence d'extrasystole.

Afin de déterminer un critère de présence fréquente ou de gravité d'extrasystole, on observe selon le procédé de l'invention les valeurs du compteur d'extrasystoles du dispositif médical implantable actif au cours des cycles cardiaques consécutifs.

D'une façon avantageuse le procédé de l'invention comprend les étapes suivantes : on détecte les signaux d'activité cardiaque dans au moins une cavité cardiaque et on détermine un cycle cardiaque ; on détecte la survenue d'extrasystoles ; on incrémente un compteur à la détection de chaque extrasystole ; et on décrémente à chaque cycle cardiaque le compteur en l'absence d'extrasystole.

Très avantageusement, on prévoit des moyens (ou, dans le procédé, une étape) pour attribuer une première pondération à chaque détection d'une extrasystole.

Dans ce cas, l'incrémentation du compteur d'extrasystoles est avantageusement mise en oeuvre avec application à la détection de chaque extrasystole de la pondération qui lui est associée, et la décrémentation de ce compteur à chaque cycle en l'absence d'extrasystole est avantageusement mise en oeuvre avec application d'une seconde pondération associée.

En outre, dans un mode préférentiel de mise en oeuvre de l'invention, le compteur d'extrasystoles ayant au moins un seuil de comptage, le dispositif (et de même pour le procédé) selon l'invention peut comprendre des moyens (une étape) d'inhibition et de déclenchement de fonctions spécifiques du dispositif médical implantable actif si l'un des seuils (d'éveil, d'activation et de désactivation) déterminés du compteur d'extrasystoles est franchi.

De plus, d'une façon avantageuse, on détermine la position de chaque extrasystole dans une salve par des moyens appropriés.

De préférence, la pondération a une valeur propre pour chaque extrasystole en fonction de sa position dans la salve et de sa fréquence d'apparition.

Une extrasystole est dite fréquente si elle est séparée de la dernière extrasystole par un minimum de cycles (typiquement 8) sans extrasystole.

Le dispositif décrit permet également d'établir un critère de présence fréquente d'extrasystole.

Dans ce cas, une extrasystole est dite fréquente si le Nombre d'extrasystoles Successives est supérieur ou égal à un.

Elle est dite non fréquente dans le cas contraire.

Dans un mode de réalisation avantageux, la pondération est réalisée par une progression de type arithmétique ou géométrique avec une modulation prédéfinie.

La modulation peut dépendre de l'arythmie particulière recherchée pour chaque fonction à inhiber ou déclencher.

De préférence, les pondérations sont des multiples entiers.

De préférence, le compteur d'extrasystoles est remis à zéro par des moyens d'initialisation.

De plus, dans un mode préféré, l'initialisation du compteur d'extrasystoles peut être déclenchée par une programmation externe du dispositif médical implantable actif et d'une façon avantageuses le compteur d'extrasystoles est limité à zéro en valeur inférieure.

D'autres caractéristiques et avantages de l'invention apparaîtront à la lecture de la description détaillée ci-dessous en référence aux dessins annexés.
La figure 1 est un diagramme schématique de réalisation préférentielle du compteur d'extrasystoles selon l'invention.
La figure 2 est une représentation des valeurs du compteur d'extrasystoles en fonction des événements apparaissant au cours des différents cycles cardiaques selon un premier exemple de réalisation préféré.
La figure 3 est une représentation des valeurs du compteur d'extrasystoles en fonction des événements apparaissant au cours des différents cycles cardiaques selon une deuxième exemple de réalisation préféré.

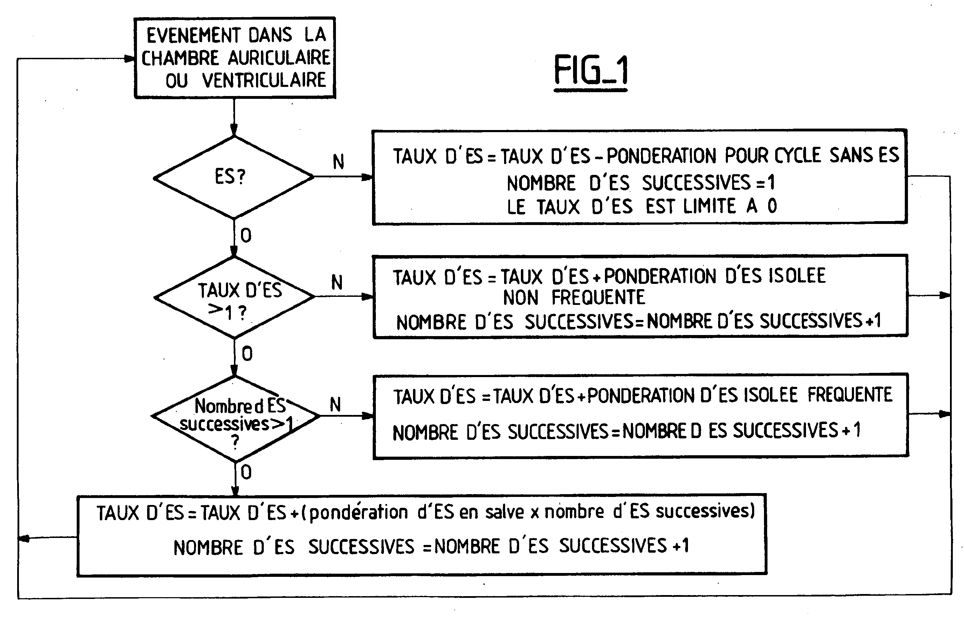

La figure 1 montre le principe de fonctionnement du compteur d'extrasystoles selon l'invention.

À l'initialisation du système le compteur d'extrasystoles est remis a zéro.

D'une façon connue en soi par l'homme du métier, on surveille et détecte par le dispositif médical implantable les événements survenant dans la chambre auriculaire A ou ventriculaire V du coeur et on détecte l'apparition d'extrasystoles.

Si une extrasystole ES isolée est déterminée, le compteur d'extrasystoles est incrémenté d'une pondération typique de 8 si l'extrasystole est considérée comme non fréquente et de 1 dans le cas contraire.

Sur les extrasystoles en salve, le compteur d'extrasystoles est incrémenté typiquement par une progression arithmétique avec une pondération déterminée, par exemple de un, multiplié par le numéro de l'extrasystole détectée.

En l'absence d'extrasystole, on décrémente à chaque cycle le compteur avec la pondération associée, typiquement de un.

Dans les figures, le Taux d'extrasystoles correspond à la valeur du compteur d'extrasystoles.

Un deuxième compteur compte le Nombre d'extrasystoles Successives indiquant ainsi le "numéro" de l'extrasystole en cours à l'intérieur d'une salve.

Sur un doublet d'extrasystoles le compteur d'extrasystoles est donc incrémenté de un à la détection du premier événement formant le doublet et de deux, par exemple, sur le deuxième événement.

D'autres pondérations sont possibles comme par exemple cinq sur le premier événement et dix sur le second.

De préférence, les pondérations sont des multiples entiers.

Mais d'une façon générale ils peuvent avoir des valeurs quelconques.

Par exemple, sur une salve d'extrasystoles, le compteur est incrémenté d'une valeur qui est un multiple du numéro de l'extrasystole à l'intérieur de la salve.

Dans un exemple préféré la pondération est un multiple de 1.

Le compteur d'extrasystoles est donc incrémenté de 1 sur le premier événement et de 2 sur le deuxième.

Pour une pondération correspondant à un multiple de 5, le compteur sera incrémenté de 5 sur le premier événement, 10 sur le second, 15 sur le troisième et ainsi de suite.

La modulation peut suivre une progression géométrique à la détection de chaque extrasystole, ou encore chaque extrasystole dans une salve peut avoir une pondération spécifique.

Sur tout autre cycle (spontané ou stimulé sans extrasystole) correspondant au recueil d'une onde P (auriculaire) ou R (ventriculaire) ou à une stimulation, le compteur d'extrasystoles est typiquement décrémenté d'une unité.

De préférence le compteur d'extrasystoles est limité à zéro en valeur inférieure.

La pondération qui est appliquée à chaque détection d'une extrasystole d'une part et à chaque cycle sans extrasystole d'autre part, ainsi que la modulation, permettent de mettre en valeur certain troubles du rythme cardiaque, désignés sous le terme de "motifs".

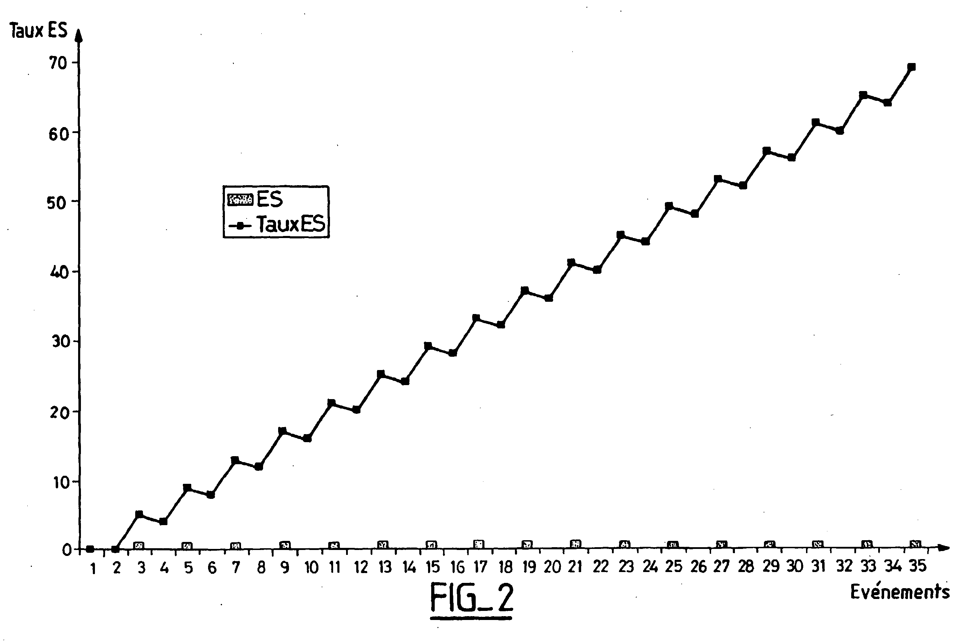

Si on recherche, par exemple, un bigéminisme (une répétition d'une extrasystole sur deux cycles cardiaques) il conviendra d'associer un poids (ou multiple) important au motif recherché comme cela est montré dans la figure 2, où la pondération correspond à un multiple de cinq.

L'invention permet donc de différencier la gravité de certains événements.

Dans l'exemple d'une valeur typique de 1 pour les cycles avec extrasystole et sans extrasystole, 10 extrasystoles isolées en 60 cycles cardiaques sont moins graves qu'une salve de 10 extrasystoles consécutives.

Dans le deuxième cas, le compteur d'extrasystoles calculera, par exemple, les valeurs 1 + 2 + 3 + 4 + 5 + 6 + 7 + 8 + 9 + 10 = 55

D'une façon plus générale, si un motif ou une répétition particulière est recherché on pondérera de façon particulière chaque élément de ce motif afin de le faire ressortir facilement au moment du comptage.

De même, la notion de fréquence d'apparition des extrasystoles pourra être révélée par un choix adéquat de la pondération et de la décrémentation du compteur.

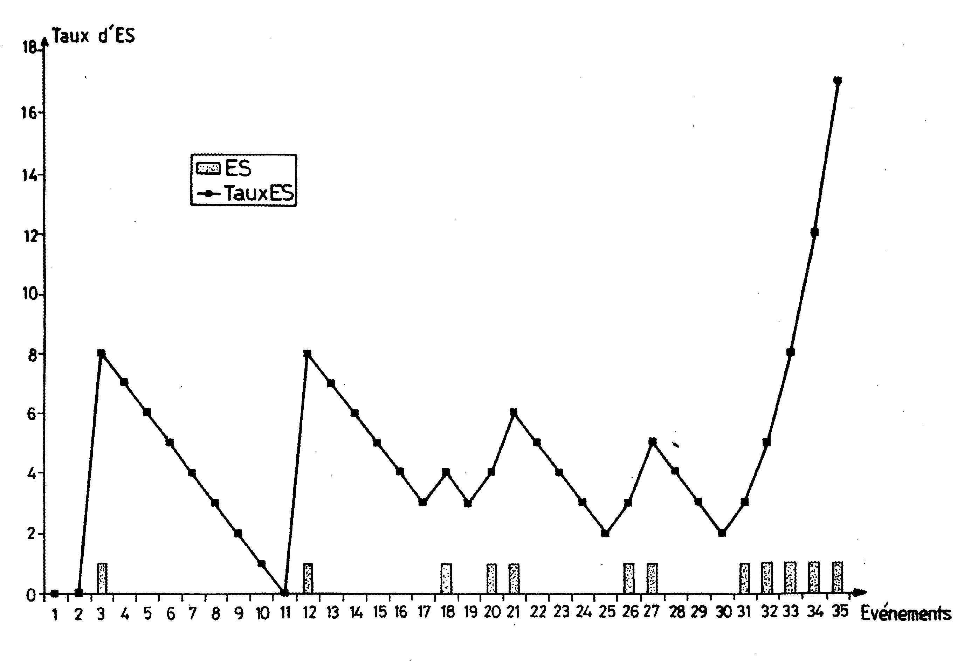

Par exemple, comme cela est illustré sur la figure 3, on peut attribuer une pondération de 8 à une extrasystole isolée ou à la première extrasystole d'une salve si elle est non fréquente et une pondération de 1 si elle est fréquente.

Ensuite, on incrémente le compteur d'extrasystoles de 2, 3, etc. à la survenue de la deuxième, troisième, etc. extrasystole respective d'une salve.

Par contre, à chaque cycle cardiaque sans survenue d'extrasystole, on décrémente le compteur d'extrasystoles de un.

Suivant les fonctions mises en oeuvre dans le dispositif, on pourra leur associer une valeur de seuil du compteur d'extrasystoles, seuil au franchissement duquel la fonction sera activée ou désactivée, selon le cas.

Elle pourra être réactivée si le seuil est franchi dans l'autre sens ou atteint une autre valeur, différente du seuil précédent (effet d'hystérésis).

La fonction de mise en éveil des fonctions de défibrillation (délivrance d'un choc à haute énergie) pourra être activée pour des valeurs importantes du compteur d'extrasystoles.

Si le compteur franchit une seconde valeur, supérieure à ce premier seuil, la fonction en-question pourra alors être déclenchée.

Tant que le compteur d'extrasystoles est en dessous de ce seuil, la fonction n'est pas mise en service, si bien qu'une économie sur le courant de la source d'énergie peut être réalisée.

Aussi, pour chaque fonction, on peut mettre en oeuvre plusieurs seuils l'activant ou la désactivant.

Par exemple, dans le mode préférentiel de mise en oeuvre de l'invention selon la figure 3, on déclenche une fonction, par exemple l'accélération du rythme de stimulation cardiaque, sur la survenue d'une extrasystole auriculaire si la valeur du compteur d'extrasystoles est supérieure ou égale à un et on inhibe la fonction si la valeur du compteur d'extrasystoles a franchi le seuil de 16, c'est-à-dire est au-dessus de ce seuil.

Dans ce dernier cas, la fonction enclenchée a échoué.

## Revendications

1. Dispositif médical implantable actif comprenant :
a) des moyens de détection des signaux d'activité cardiaque dans au moins une cavité cardiaque et des moyens de détermination du cycle cardiaque,
b) des moyens de détection de la survenue d'extrasystoles,
c) un compteur d'extrasystoles,
d) des moyens d'incrémentation du compteur d'extrasystoles à la détection de chaque extrasystole,
e) des moyens de décrémentation à chaque cycle cardiaque du compteur d'extrasystoles en l'absence d'extrasystole.

2. Dispositif selon la revendication 1, **caractérisé en ce qu'**il comprend en outre des moyens d'attribution d'une pondération à chaque détection d'une extrasystole.

3. Dispositif selon la revendication 2, **caractérisé en ce que** les moyens d'incrémentation du compteur d'extrasystoles appliquent à la détection de chaque extrasystole la pondération qui lui est associée.

4. Dispositif selon la revendication 1, **caractérisé en ce que** les moyens de décrémentation à chaque cycle cardiaque du compteur d'extrasystoles en l'absence d'extrasystole appliquent une pondération associée.

5. Dispositif selon la revendication 2, **caractérisé en ce qu'**il comprend en outre des moyens pour déterminer la position d'une extrasystole dans une salve.

6. Dispositif selon l'une des revendications précédentes, **caractérisé en ce que**, le compteur d'extrasystoles ayant au moins un seuil de comptage, il comprend en outre des moyens d'inhibition et de déclenchement de fonctions spécifiques du dispositif médical implantable actif si l'un des seuils déterminés du compteur d'extrasystoles est franchi.

7. Dispositif selon l'une des revendications 2 à 5, **caractérisé en ce que** la pondération a une valeur propre pour chaque extrasystole en fonction de sa position dans la salve et de la fréquence d'apparition des extrasystoles.

8. Dispositif selon la revendication 7, **caractérisé en ce que** la pondération est réalisée par une progression de type arithmétique ou géométrique avec une modulation prédéfinie.

9. Dispositif selon l'une des revendications 2 à 6, **caractérisé en ce que** les pondérations sont des multiples entiers.

10. Dispositif selon l'une des revendications précédentes, **caractérisé en ce que** des moyens d'initialisation du compteur d'extrasystoles sont prévus pour initialiser à zéro le compteur d'extrasystoles.

11. Dispositif selon l'une des revendications précédentes, **caractérisé en ce que** des moyens sont prévus pour déclencher l'initialisation du compteur d'extrasystoles par une programmation externe du dispositif médical implantable actif.

12. Dispositif selon l'une des revendications précédentes, **caractérisé en ce que** le compteur d'extrasystoles est limité à zéro en valeur inférieure.

13. Procédé de détermination d'un critère de présence fréquente ou de gravité d'extrasystole pour la commande d'un dispositif médical implantable actif comprenant les étapes suivantes :
a) on détecte les signaux d'activité cardiaque dans au moins une cavité cardiaque et on détermine un cycle cardiaque,
b) on détecte la survenue d'extrasystoles,
c) on incrémente un compteur d'extrasystoles à la détection de chaque extrasystole,
d) on décrémente à chaque cycle cardiaque le compteur d'extrasystoles en l'absence d'extrasystole.

14. Procédé selon la revendication 13, **caractérisé en ce que** l'on attribue une pondération à chaque détection d'une extrasystole.

15. Procédé selon la revendication 14, **caractérisé en ce que** l'étape d'incrémentation du compteur d'extrasystoles est opérée avec application à la détection de chaque extrasystole de la pondération qui lui est associée.

16. Procédé selon la revendication 13, **caractérisé en ce que** l'étape de décrémentation à chaque cycle cardiaque du compteur d'extrasystoles est opérée avec application à la détection de chaque extrasystole de la pondération qui lui est associée.

17. Procédé selon la revendication 14, **caractérisé en ce qu'**il comprend en outre l'étape suivante :
f) on détermine la position de chaque extrasystole dans une salve.

18. Procédé selon l'une des revendications 13 à 17, **caractérisé en ce que**, le compteur d'extrasystoles ayant au moins un seuil de comptage, il comprend en outre l'étape suivante :
e) on inhibe ou on déclenche des fonctions spécifiques du dispositif médical implantable actif si l'un des seuils déterminés du compteur d'extrasystoles est franchi.

## Claims

1. An active implantable medical device comprising:
a) means for detecting signals of cardiac activity in at least one cardiac cavity and means for determining the cardiac cycle,
b) means for detecting the occurrence of extrasystoles,
c) an extrasystole counter;
d) means for incrementing the extrasystole counter on detection of each extrasystole,
e) means for decrementing the extrasystole counter at each cardiac cycle in the absence of an extrasystole.

2. The device according to claim 1, **characterised in that** it further comprises means for attributing a weighting at each detection of an extrasystole.

3. The device according to claim 2, **characterised in that** the means for incrementing the extrasystole counter applies, on detection of each extrasystole, the weighting that is associated with it.

4. The device according to claim 1, **characterised in that** the means for decrementing the extrasystole counter at each cardiac cycle in the absence of an extrasystole applies an associated weighting.

5. The device according to claim 2, **characterised in that** it further comprises means for determining the position of an extrasystole in a salvo.

6. The device according to one of the preceding claims, **characterised in that**, the extrasystole counter having at least one counting threshold, it further comprises means for inhibiting and initiating specific functions of the active implantable medical device if one of the determined thresholds of the extrasystole counter is crossed.

7. The device according to one of claims 2 to 5, **characterised in that** the weighting has its own value for each extrasystole according to its position in the salvo and the frequency of occurrence of the extrasystoles.

8. The device according to claim 7, **characterised in that** the weighting is implemented as an arithmetical or a geometrical progression with a predetermined modulation.

9. The device according to one of claims 2 to 6, **characterised in that** the weightings are integer multiples.

10. The device according to one of the preceding claims, **characterised in that** there is provided a means for initialising the extrasystole counter in order to set the extrasystole counter to zero.

11. The device according to one of the preceding claims, **characterised in that** there is provided a means for initiating the initialisation of the extrasystole counter by a programming external to the active implantable medical device.

12. The device according to one of the preceding claims, **characterised in that** the extrasystole counter is limited to a lowest value of zero.

13. A process for the determination of a criterion of a frequent presence or gravity of extrasystoles for the control of an active implantable medical device, comprising the following steps:
a) detecting cardiac activity signals in at least one cardiac cavity and determining a cardiac cycle,
b) detecting the occurrence of extrasystoles,
c) incrementing an extrasystole counter on detection of each extrasystole,
d) decrementing the extrasystole counter at each cardiac cycle in the absence of an extrasystole.

14. The process according to claim 13, **characterised by** attributing a weighting at each detection of an extrasystole.

15. The process according to claim 14, **characterised in that** the step of incrementing the extrasystole counter is executed by applying, on detection of each extrasystole, the weighting that is associated with it.

16. The process according to claim 13, **characterised in that** the step of decrementing the extrasystole counter at each cardiac cycle is executed by applying, on detection of each extrasystole, the weighting that is associated with it.

17. The process according to claim 14, **characterised in that** it further comprises the following step:
f) determining the position of each extrasystole in a salvo.

18. The process according to one of claims 13 to 17, **characterised in that**, the extrasystole counter having at least one counting threshold, it further comprises the following step:
e) inhibiting or initiating specific functions of the active implantable medical device if one of the determined thresholds of the extrasystole counter is crossed.

## Patentansprüche

1. Aktive implantierbare medizinische Vorrichtung, mit:
a) Mitteln zur Erfassung der Signale der Herzaktivität in mindestens einer Herzkammer und Mitteln zur Bestimmung des Herzzyklus,
b) Mitteln zur Erfassung des Auftretens von Extrasystolen,
c) einem Extrasystolen-Zähler,
d) Mitteln zur Inkrementierung des Extrasystolen-Zählers bei der Erfassung einer jeden Extrasystole,
e) Mitteln zur Dekrementierung des Extrasystolen-Zählers bei jedem Herzzyklus bei Abwesenheit einer Extrasystole.

2. Vorrichtung nach Anspruch 1, **dadurch gekennzeichnet, dass** sie außerdem Mittel zur Zuweisung einer Gewichtung bei jeder Erfassung einer-Extrasystole umfasst.

3. Vorrichtung nach Anspruch 2, **dadurch gekennzeichnet, dass** die Mittel zur Inkrementierung des Extrasystolen-Zählers bei der Erfassung einer jeden Extrasystole die ihr zugeordnete Gewichtung anwenden.

4. Vorrichtung nach Anspruch 1, **dadurch gekennzeichnet, dass** die Mittel zur Dekrementierung des Extrasystolen-Zählers bei jedem Herzzyklus bei Abwesenheit einer Extrasystole eine zugeordnete Gewichtung anwenden.

5. Vorrichtung nach Anspruch 2, **dadurch gekennzeichnet, dass** sie außerdem Mittel zur Bestimmung der Position einer Extrasystole in einer Salve umfasst.

6. Vorrichtung nach einem der vorherigen Ansprüche, **dadurch gekennzeichnet, dass**, wobei der Extrasystolen-Zähler mindestens eine Zählschwelle aufweist, sie außerdem Mittel zur Unterdrückung und zur Auslösung spezifischer Funktionen der aktiven implantierbaren medizinischen Vorrichtung umfasst, wenn eine der bestimmten Schwellen des Extrasystolen-Zählers durchschritten wird.

7. Vorrichtung nach einem der Ansprüche 2 bis 5, **dadurch gekennzeichnet, dass** die Gewichtung für jede Extrasystole einen eigenen Wert hat, in Abhängigkeit ihrer Position innerhalb der Salve und der Frequenz des Auftretens der Extrasystolen.

8. Vorrichtung nach Anspruch 7, **dadurch gekennzeichnet, dass** die Gewichtung durch eine arithmetische oder geometrische Folge mit einer vorbestimmten Modulation verwirklicht ist.

9. Vorrichtung nach einem der Ansprüche 2 bis 6, **dadurch gekennzeichnet, dass** die Gewichtungen ganze Vielfache sind.

10. Vorrichtung nach einem der vorherigen Ansprüche, **dadurch gekennzeichnet, dass** Mittel zur Initialisierung des Extrasystolen-Zählers vorgesehen sind, um den Extrasystolen-Zähler auf Null zu setzen.

11. Vorrichtung nach einem der vorherigen Ansprüche, **dadurch gekennzeichnet, dass** Mittel zur Auslösung der Initialisierung des Extrasystolen-Zählers durch eine Programmierung außerhalb der aktiven implantierbaren medizinischen Vorrichtung vorgesehen sind.

12. Vorrichtung nach einem der vorherigen Ansprüche, **dadurch gekennzeichnet, dass** der Extrasystolen-Zähler nach unten auf den Wert null begrenzt ist.

13. Verfahren zur Bestimmung eines Kriteriums des häufigen Auftretens oder der Schwere von Extrasystolen zur Steuerung einer aktiven implantierbaren medizinischen Vorrichtung mit den folgenden Schritten:
a) man erfasst die Signale der Herzaktivität in mindestens einer Herzkammer und man bestimmt einen Herzzyklus,
b) man erfasst das Auftreten von Extrasystolen,
c) man inkrementiert einen Extrasystolen-Zähler bei der Erfassung einer jeden Extrasystole,
d) man dekrementiert den Extrasystolen-Zähler bei jedem Herzzyklus bei Abwesenheit einer Extrasystole.

14. Verfahren nach Anspruch 13, **dadurch gekennzeichnet, dass** man eine Gewichtung bei jeder Erfassung einer Extrasystole zuweist.

15. Verfahren nach Anspruch 14, **dadurch gekennzeichnet, dass** der Schritt der Inkrementierung des Extrasystolen-Zählers bei Anwendung, bei der Erfassung einer jeden Extrasystole, der ihr zugeordneten Gewichtung durchgeführt wird.

16. Verfahren nach Anspruch 13, **dadurch gekennzeichnet, dass** der Schritt der Dekrementierung des Extrasystolen-Zählers bei jedem Herzzyklus bei Anwendung, bei der Erfassung einer jeden Extrasystole, der ihr zugeordneten Gewichtung durchgeführt wird.

17. Verfahren nach Anspruch 14, **dadurch gekennzeichnet, dass** es außerdem den folgenden Schritt umfasst:
f) man bestimmt die Position einer jeden Extrasystole in einer Salve.

18. Verfahren nach einem der Ansprüche 13 bis 17, **dadurch gekennzeichnet, dass**, wobei der Extrasystolen-Zähler mindestens eine Zählschwelle aufweist, es außerdem den folgenden Schritt umfasst:
e) man unterdrückt oder man löst spezifische Funktionen der aktiven implantierbaren medizinischen Vorrichtung aus, wenn eine der bestimmten Schwellen des Extrasystolen-Zählers durchschritten wird.
